(19) Europäisches Patentamt
European Patent Office
Office européen des brevets

(11) Veröffentlichungsnummer: **0 319 862 B1**

(12) # EUROPÄISCHE PATENTSCHRIFT

(45) Veröffentlichungstag der Patentschrift: **12.01.94**

(51) Int. Cl.$^5$: **B01D 71/00**

(21) Anmeldenummer: **88120194.1**

(22) Anmeldetag: **03.12.88**

(54) **Biocompatible Dialysemembran aus Cellulose mit erhöhter Beta-2-Microglobulinadsorption.**

(30) Priorität: **11.12.87 DE 3742072**

(43) Veröffentlichungstag der Anmeldung:
**14.06.89 Patentblatt 89/24**

(45) Bekanntmachung des Hinweises auf die Patenterteilung:
**12.01.94 Patentblatt 94/02**

(84) Benannte Vertragsstaaten:
**DE ES FR IT NL**

(56) Entgegenhaltungen:
**EP-A- 0 236 509**
**EP-A- 0 247 592**
**EP-A- 0 254 025**

(73) Patentinhaber: **Akzo N.V.**
**Postbus 9300**
**Velperweg 76**
**NL-6800 SB Arnhem(NL)**

(72) Erfinder: **Diamantoglou, Michael, Dr.**
**Kolpingstrasse 4**
**D-8765 Erlenbach(DE)**
Erfinder: **Kuhne, Helmut, Dr.**
**Südstrasse 17**
**D-5166 Kreuzau(DE)**

(74) Vertreter: **Fett, Günter**
**Akzo Patente GmbH,**
**Postfach 10 01 49**
**D-42097 Wuppertal (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.9/3.3.3)

## Beschreibung

Die Erfindung betrifft eine Dialysemembran für die Hämodialyse in Form von Flachfolien, Schlauchfolien oder Hohlfäden aus durch Substitution modifizierter Cellulose.

Dialysemembranen aus Cellulose für die Hämodialyse in Form von Flachfolien, Schlauchfolien oder Hohlfäden sind bereits seit längerem bekannt und werden nach wie vor bevorzugt in künstlichen Nieren eingesetzt. Einige Beschwerden verursachende Eigenschaften ließen sich jedoch noch nicht beseitigen.

So ist aus der DE-C-27 05 735 eine Dialysemembran für die Hämodialyse mit daran chemisch gebundenen antithrombogenen Verbindungen bekannt, wobei die Dialysemembran aus zwei oder mehreren Schichten einer aus Cuoxamcelluloselösungen regenerierten Cellulose besteht, die jeweils aus getrennt gespeisten Schlitzen einer Spinndüse erhalten worden ist, die antithrombogene Wirkstoffe chemisch gebunden enthält.

Es ist aber auch bereits in der DE-A-17 20 087 vorgeschlagen worden, dadurch daß das Polymermaterial der Membran mit einem Alkylhalogenid umgesetzt und danach das erhaltene Material mit einem Alkalisalz einer antithrombogenen Verbindung mit kationischem Rest (z.B. Heparin oder eine Heparinoidverbindung) umgesetzt wird, die Gefahr der Gerinnung des Blutes zu verringern. Zu den möglichen Alkylhalogeniden werden dabei auch Halogenalkyldialkylamine gerechnet. Auch Cellulose, jedoch im wesentlichen Celluloseacetat, zählt zu den möglichen Polymeren.

Eine antithrombogene Wirkung dieser bekannten Dialysemembranen wird nur beobachtet, wenn der Substitutionsgrad der modifizierten Cellulose hoch ist, d.h. größer als mindestens 0,1 und in einem gesonderten Schritt eine Vorheparinisierung mit relativ hoher Heparinkonzentration (0,1 bis 1 Gew.%-ige Lösungen) durchgeführt wird.

Aus der DE-A-35 24 596 ist bereits eine Dialysemembran mit verbesserter Biocompatibilität bekannt, die sich dadurch auszeichnet, daß der mittlere Substitutionsgrad einer modifizierten Cellulose 0,02 bis 0,07 beträgt. Vorzugsweise enthält die bekannte Dialysemembran aus modifizierter Cellulose solche modifizierte Cellulose, die eine durch die Formel

Cellulose-R′-X-Y

wiedergegebene Struktur aufweist, wobei

X für -NR″- und/oder

$$\overset{\oplus}{-NR''_2-}$$

und/oder -S-und/oder -SO- und/oder $-SO_2-$ und/oder

$$-\underset{\underset{O}{\|}}{C}-\underset{\underset{R}{|}}{N}-$$

und/oder -CO-O- und/oder -O-,

Y für -R und/oder $-NR_2$ und/oder $-Si(OR'')_3$ und/oder $-SO_3H$ und/oder -COOH und/oder $-PO_3H_2$ und/oder

$$\overset{\oplus}{-NHR_2}$$

bzw. deren Salze,

R′ für eine Alkylengruppe und/oder Cycloalkylengruppe und/oder Arylengruppe mit insgesamt 1 bis 25 C-Atomen,

R″ für ein Wasserstoffatom oder R und

R für eine Alkylgruppe mit 1 bis 5 C-Atomen und/oder eine Cycloalkylgruppe und/oder Arylgruppe steht.

Diese bekannte Dialysemembran war bereits in der Lage, Blutgerinnung, Leucopenie und Komplementaktivierung in erheblichem Umfange zu reduzieren. Eine Adsorption von Beta-2-Mikroglobulin konnte jedoch in nennenswertem Umfange nicht festgestellt werden.

Neben dem Umstand, daß Dialysemembranen aus synthetischen bzw. natürlichen Polymeren bei ihrem Einsatz in künstlichen Nieren sehr leicht eine Gerinnung des Blutes hervorrufen können, die durch entsprechende medikamentöse Behandlung weitgehend verhindert wird, tritt bei Dialysemembranen aus regenerierter Cellulose häufig bei der Behandlung eines Nierenkranken mit Dialysatoren mit Cellulose-Membranen in der ersten Zeit der Dialysebehandlung ein vorübergehender Leukozytenabfall auf. Dieser Effekt wird als Leukopenie bezeichnet.

Leukopenie ist eine Erniedrigung der Leukozytenzahl (weiße Blutkörper) im Blutkreislauf. Die Zahl der weißen Blutkörper beim Menschen beträgt ca. 4000 bis 12000 Zellen/$mm^3$.

Die Leukopenie bei der Dialyse ist am stärksten ausgeprägt 15 bis 20 Min. nach Beginn, wobei die Neutrophilen (das sind die mit neutralen oder gleichzeitig mit sauren und basischen Farbstoffen anfärbbaren Leukozyten) fast vollständig verschwinden können. Danach erholt sich die Zahl der Leukozyten innerhalb etwa einer Stunde wieder auf fast den Ausgangswert oder übersteigt diesen.

Wird nach Erholung der Leukozyten ein neuer Dialysator angeschlossen, tritt wieder Leukopenie im gleichen Ausmaß ein.

Cellulose-Membranen verursachen eine ausgeprägte Leukopenie. Auch wenn die klinische Bedeutung der Leukopenie wissenschaftlich nicht geklärt ist, besteht doch der Wunsch nach einer Dialysemembran für die Hämodialyse, die den Effekt der Leukopenie nicht zeigt, ohne daß dadurch die anderen sehr erwünschten Eigenschaften von Dialysemembranen aus regenerierter Cellulose beeinträchtigt werden.

Bei der Hämodialyse mittels Membranen aus regenerierter Cellulose hat man neben der Leukopenie auch eine deutliche Komplement-Aktivierung festgestellt. Das Komplement-System innerhalb des Blutserums ist ein komplexes, aus vielen Komponenten bestehendes Plasmaenzym-System, das auf verschiedene Weise der Abwehr von Schädigungen durch eindringende fremde Zellen (Bakterien u.a.) dient. Wenn Antikörper gegen den eindringenden Organismus vorhanden sind, kann komplementspezifisch durch den Komplex der Antikörper mit antigenen Strukturen der Fremdzellen aktiviert werden, anderenfalls erfolgt auf einem Alternativ-Weg durch besondere Oberflächenmerkmale der Fremdzellen die Komplement-Aktivierung. Das Komplement-System beruht auf einer Vielzahl von Plasma-Proteinen. Nach Aktivierung reagieren diese Proteine spezifisch in einer bestimmten Reihenfolge miteinander und am Ende wird ein zellschädigender Komplex gebildet, der die Fremdzelle zerstört.

Aus einzelnen Komponenten werden Peptide freigesetzt, die Entzündungserscheinungen auslösen und gelegentlich auch unerwünschte pathologische Folgen für den Organismus haben können. Es wird angenommen, daß die Aktivierung bei Hämodialysemembranen aus regenerierter Cellulose über den alternativen Weg erfolgt. Objektiv festgestellt werden diese Komplement-Aktivierungen durch eine Bestimmung der Komplement-Fragmente C3a und C5a.

In diesem Zusammenhang wird auf folgende Arbeiten hingewiesen: D.E. Chenoweth et al., Kidnev International Vol. 24, Seite 764 ff, 1983 und D.E. Chenoweth, Asaio-Journal Vol. 7, Seite 44 ff, 1984.

Das Carpal-Tunnel-Syndrom wird durch die bekannten modifizierten Dialysemembranen nur wenig beeinflußt. Deshalb besteht ein erhebliches Bedürfnis nach weiteren Modifizierungen der Cellulose, um auch dieses Phänomen auszuschalten.

Aufgabe der vorliegenden Erfindung war es, Dialysemembranen für die Hämodialyse in Form von Flachfolien, Schlauchfolien oder Hohlfäden aus durch Substitution modifizierter Cellulose zur Verfügung zu stellen, die hinsichtlich der Leukopenie, der Komplementaktivierung und der Blutgerinnung optimale Eigenschaften aufweisen und darüber hinaus das für den Carpal-Tunnel-Effekt verantwortliche Beta-2-Microglobulin in erheblichem Umfang zu adsorbieren und damit auch diese Beeinträchtigung bei der Hämodialyse zu unterbinden in der Lage sind.

Gelöst wird diese Aufgabe durch eine Dialysemembran, die dadurch gekennzeichnet ist, daß die modifizierte Cellulose eine durch die Formel

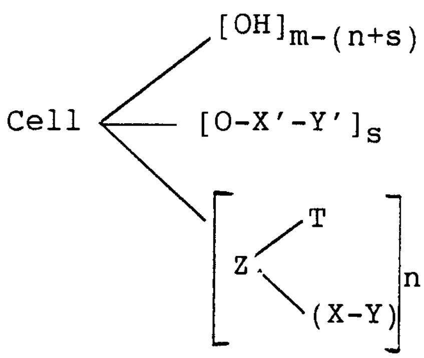

wiedergegebene Struktur aufweist, worin Cell das Gerüst des unmodifizierten Cellulosemoleküls oder des Chitinmoleküls jeweils ohne Hydroxylgruppen, Z ein N- oder S-Atom ist und für N T ein Wasserstoffatom oder X-Y bedeutet und für S T entfällt und (n + s) den mittleren Substitutionsgrad angibt mit $0 < n < m$ und $0 \leqq s < m$ und $n + s < m$ und m beim unmodifizierten Cellulosemolekül 3 und beim Chitinmolekül 2 beträgt und worin gegebenenfalls - X - entfallen kann oder

-X- und -X'- einen gegebenenfalls substituierten Alkylen-Rest (gerad-kettig und/oder verzweigt, wobei die Kohlenstoffkette auch durch Heteroatome wie O, S, N unterbrochen sein kann) und/oder Arylalkylen-, Aryl-Rest (ggf. mit Heteroatomen und/oder substituiert)

-Y und -Y' -H, und/oder
$-NR_2$ und/oder -COOH auch als Salz und/oder $-SO_3H$ auch als Salz und/oder -OR bedeuten,

wobei R ein Wasserstoffatom und/oder eine gegebenenfalls substituierte Alkylgruppe (gerad-kettig und/oder verzweigt, wobei die Kohlenstoffkette auch durch Heteroatome wie O, S, N unterbrochen sein kann) und/oder Aryl- und/oder Arylalkyl- Rest (ggf. mit Heteroatomen und/oder substituiert) bedeutet

und X gleich oder verschieden von X' und Y gleich oder verschieden von Y' ist und die maximale Anzahl der C-Atome in X-Y bzw. X'-Y' 12 beträgt.

Während unmodifizierte Cellulose 3 für eine Substitution zur Verfügung stehende Hydroxylgruppen enthält, ist bei Chitin bereits eine Hydroxylgruppe durch Acetamidgruppen substituiert. Diese Substitution wird im Rahmen der vorliegenden Erfindung beim mittleren Substitutionsgrad nicht mehr berücksichtigt, sofern modifizierte Cellulosen auf der Basis des Chitinmolekül-Gerüstes eingesetzt werden.

Die erfindungsgemäßen Dialysemembranen lassen sich aus substituierter Cellulose im Gemisch mit unmodifizierter Cellulose auf den gewünschten Substitutionsgrad einstellen. Die Substitution erfolgt nach an sich bekannten Verfahren. Besonders bevorzugt sind solche Dialysemembranen, bei denen der Substitutionsgrad n = 0,02 bis 0,30 beträgt. Der Substitutionsgrad s beträgt in bevorzugter Weise 0,03 bis 0,12.

Das in der US-A-3 702 754 beschriebene Verfahren ist auch geeignet, wenn darauf geachtet wird, daß der Abbau begrenzt bleibt. Sofern bei diesem Verfahren eine Vernetzung eintritt, sind die Produkte zum Teil in den bekannten Celluloselösungsmitteln, beispielsweise Cuoxam, unlöslich, so daß sie für die erfindungsgemäße Dialysemembran dann ungeeignet sind.

Andere bekannte Verfahren haben sich jedoch als besser geeignet erwiesen. Beispielsweise kann hierzu auf Journal of Polymer Science - Part C, No. 11, (1965), Seiten 107 - 118 oder J. Am. Chem. Soc. Febr. 1950, Seiten 670 - 674 und auf "Cellulose and Cellulose Derivatives" Part II, herausgegeben von Ott, Spurlin und Grafflin, Interscience Publishers, Inc., New York, 2. Auflage, 1954, Seite 822 verwiesen werden. Bei diesen Verfahren wird der Substitutionsgrad beliebig hoch eingestellt.

Im Rahmen der vorliegenden Erfindung wurde die Komplement-Aktivierung anhand der Fragmente C3a bzw. C5a beurteilt. Dazu wurden in vitro 300 ml heparinisiertes Blutplasma über einen Zeitraum von 4 Std. mit einem Plasmafluß von 100 ml/min. durch einen Dialysator mit 1 $m^2$ effektiver Austauschfläche rezirkuliert. In dem Plasma wurden die C3a-Fragmente mit Hilfe der RIA-Methode (Upjohn-Test) bestimmt. Die relative Komplement-Aktivierung für den jeweiligen Meßzeitpunkt wurde durch Bildung des Verhältnisses der Konzentration zum Zeitpunkt der Probenahme mit dem Anfangswert in Prozent errechnet. Zur Bewertung wurde der Meßwert nach 4 Std. Rezirkulationszeit herangezogen. Flachmembranen werden mit heparinisiertem Blutplasma 3 Stunden inkubiert und anschließend die C3a-Fragmente bestimmt. Die C5a-Fragmente wurden analog bestimmt.

Die Erhöhung des beta-2-Mikroglobulinspiegels bei Langzeit-Dialysepatienten wird nach Verwendung von Membranen aus regenerierter Cellulose beobachtet und wird darauf zurückgeführt, daß diese Membranen für Stoffe im Molekularbereich von 1000 bis 20.000 weniger durchlässig sind und letztere deshalb bei der Dialyse nicht in ausreichendem Maße entfernt werden. An die üblichen Membranen aus regenerierter Cellulose adsorbiert sich das beta-2-Mikroglobulin nicht in nennenswertem Umfang. Hierzu aber können in unerwarteter Weise die erfindungsgemäßen Cellulosederivate beitragen.

Gemessen wird im Rahmen der Erfindung der beta-2-Mikroglobulingehalt, der an die Membran adsorbiert wird, auf folgende Weise:
In je 500 mg Substanz (Dialysemembran) werden 10 ml Humanblutplasma gegeben und 30 min. bei 37 °C inkubiert. Das Humanblutplasma hat einen Gehalt an beta-2-Mikroglobulin von 13,67 mg/l. Die Probe wird bei 3000 Upm 15 min. zentrifugiert. Im Überstand wird der Gehalt an beta-2-Mikroglobulin festgestellt. Anschließend wird die Probe 2 x mit je 10 ml Phosphat-buffer-saline gewaschen. In den Waschflüssigkeiten wird der Mikroglobulingehalt ebenfalls festgestellt. Aus der Differenz zwischen ursprünglichem und nicht absorbiertem beta-2-Mikroglobulin läßt sich die prozentuale Menge an absorbiertem beta-2-Mikroglobulin errechnen.

Der Durchschnittspolymerisationsgrad DP wurde in einer Cuen-Lösung nach DIN 54270 bestimmt.

Der Verätherungsgrad und/oder Veresterungsgrad wurden anhand der Analysenergebnisse bestimmt, die für die Substituenten bekannt und typisch sind, beispielsweise Stickstoff nach Kjeldahl, Schwefel nach Schöniger oder Phosphor nach der Molybdatmethode, gegebenenfalls aus der Differenz vor und nach einer Verseifung.

Gut geeignete Dialysemembranen ergeben sich bei modifizierten Cellulosen, sofern gegebenenfalls von substituierten X- bzw.X'-Molekülresten ausgegangen wird, wenn X bzw. X' mit den Molekülresten Y bzw. Y' substituiert sind.

Im allgemeinen sind solche Dialysemembranen bevorzugt, bei denen die Substituenten Y bzw. Y' sekundäre, tertiäre oder quaternäre Aminogruppen, Carboxygruppen und/oder Sulfogruppen sind.

Gute Ergebnisse hinsichtlich der angestrebten Biocompatibilität erhält man mit Dialysemembranen mit modifizierter Cellulose, bei denen in der angegebenen Strukturformel -[ XY ] Dialkylaminoalkylen, Carboxyalkylen, Carboxyarylalkylen und/oder Sulfoalkylen bedeutet.

Alkylreste für R sind vorzugsweise Methylgruppen, Äthylgruppen und/oder Propylgruppen.

Seit vielen Jahren haben sich Dialysemembranen aus aus Cuoxamlösungen regenerierter Cellulose aufgrund ihres hohen Entwicklungsstandes bewährt.
Im Rahmen der Erfindung ist es aber auch ohne Einschränkung möglich, die Regeneration aus anderen Celluloselösungsmitteln durchzuführen, beispielsweise aus Dimethylacetamid/LiCl oder aus tertiären Aminoxiden/Wasser oder auch aus Viskoselösungen. Modifizierte Cellulosen mit einem Chitinmolekülgerüst lassen sich aus Cuoxamlösungen wegen ihrer Unlöslichkeit nicht regenerieren. Aus den anderen genannten Celluloselösungsmitteln ist dieses jedoch problemlos möglich.

Beispiel 1

**A) Umsetzung von Cellulose mit p-Toluolsulfonsäurechlorid**

In einem 10 l-Kolben wurden 486 g (3 Mol) Cellulose (DP = 1400, gemessen im Lösungsmittel Cuen) in 6000 ml Pyridin (wasserfrei) suspendiert. Danach wurden 171,45 g (0,9 Mol) p-Toluolsulfonsäurechlorid hinzugefügt und die Mischung 48 Stunden bei 25 °C gerührt. Das Reaktionsprodukt wurde abgesaugt, nacheinander mit Äthanol, Wasser und Äthanol gewaschen und im Vakuumtrockenschrank bei 65 °C getrocknet. Dabei wurden 565 g eines Produktes mit einem Schwefelgehalt von 3,03 %, entsprechend einem Veresterungsgrad von 0,18, erhalten.

**B) Umsetzung von Cellulosetoluolsulfonsäureester mit 2-Mercaptobernsteinsäure**

In einem Mischer wurden 150 ml Äthanol und 27 g (0,18 Mol) 2-Mercaptobernsteinsäure vorgelegt und mit einer Lösung von 50,4 g (0,90 Mol) Kaliumhydroxid in 150 ml Wasser neutralisiert. Danach wurden 189,72 g (1 Mol) des zuvor erhaltenen Esters (Beispiel 1A) hinzugefügt und das Gemisch 18 Stunden bei 80 °C erhitzt. Das resultierende Produkt wurde mit Wasser, wäßriger Salzsäure-Lösung und Äthanol gewaschen und im Vakuumtrockenschrank bei 65 °C getrocknet. Dabei wurden 173 g eines Produktes mit einem Schwefelgehalt von 1,65%, entsprechend einem DS (n) von 0,09, erhalten.

Aus diesem Cellulosederivat wurde nach üblicher Verfahrensweise eine Cuoxam-Lösung mit 9 % Cellulosederivat-Gehalt hergestellt und zu Kapillarmembranen versponnen.

Die auf diese Weise erhaltenen Cellulosederivatmembranen wiesen folgende Eigenschaften auf:

| | |
|---|---|
| Wanddicke: | 10 µm |
| Innendurchmesser: | 200 µm |
| Ultrafiltrationsrate: | 4,6 ml/h•m$^2$•mm Hg bei 37 °C |
| Vitamin B12-Permeabilität: | 4,9•10$^{-3}$ cm/min bei 37 °C |
| Beta-2-Microglobulinadsorption: | 35 % |

Die obengenannte Cellulosederivatmembran weist im Vergleich zu unmodifizierten Cellulose-Membranen eine geringere Komplementaktivierung auf. Gegenüber der unmodifizierten Cellulosemembran beträgt die C3a-Reduzierung 96 %.

Beispiel 2

In einem Mischer wurden 54 g (0,72 Mol) Aminoessigsäure und 200 ml Wasser vorgelegt. Nach der Neutralisation der Aminosäure mit einer Lösung von 50,4 g (0,9 Mol) Kaliumhydroxid in 100 ml Wasser wurden 189,72 g (1 Mol) des Esters vom Beispiel 1A hinzugefügt und das Gemisch 18 Stunden bei 80 °C erhitzt. Das Reaktionsprodukt wurde mit Wasser und Äthanol gewaschen und im Vakuumtrockenschrank bei 65 °C getrocknet.

| | |
|---|---|
| Ausbeute: | 167 g |
| N-Gehalt: | 0,7% |
| Substitutionsgrad(n): | 0,085 |

Die aus einer Cuoxam-Lösung nach üblicher Verfahrensweise hergestellten Kapillarmembranen wiesen folgende Eigenschaften auf:

| | |
|---|---|
| Wanddicke: | 11 µm |
| Innendurchmesser: | 205 µm |
| Ultrafiltrationsrate: | 4,1 ml/h•m$^2$•mm Hg bei 37 °C |
| Vitamin B12-Permeabilität: | 4,5•10$^{-3}$ cm/min bei 37 °C |
| Beta-2-Microglobulinadsorption: | 28 % |

Im Vergleich zu unmodifizierter Cellulose beträgt die C3a-Reduktion 92 %.

Beispiel 3

Analog dem Beispiel 1A wurde durch Umsetzung von niedrigsubstituierter Diäthylaminoäthylcellulose mit p-Toluolsulfonsäurechlorid Diäthylaminoäthyl-cellulose-p-toluolsulfonsäureester mit folgender Spezifikation hergestellt.

| | |
|---|---|
| Verätherungsgrad(s): | 0,04 |
| Veresterungsgrad: | 0,10 |

Analog dem Beispiel 1B wurde durch Umsetzung des Esters mit 3-Mercaptopropionsäure in Anwesenheit von KOH ein Cellulosederivat mit einem S-Gehalt von 0,94 % (n = 0,05) und einem N-Gehalt von 0,3 % (s = 0,037) erhalten.

Die aus einer Cuoxam-Lösung nach üblicher Verfahrensweise hergestellten Kapillarmembranen wiesen folgende Eigenschaften auf:

| | |
|---|---|
| Wanddicke: | 14 µm |
| Innendurchmesser: | 205 µm |
| Ultrafiltrationsrate: | 4,2 ml/h•m$^2$•mm Hg bei 37 °C |
| Vitamin B12-Permeabilität: | 4,8•10$^{-3}$ cm/min bei 37 °C |
| Beta-2-Microglobulinadsorption: | 40 % |

Im Vergleich zu unmodifizierter Cellulose beträgt die C3a-Reduktion 98 %.

Beispiele 4 - 9

Analog dem Beispiel 1 oder Beispiel 2 wurden die in der Tabelle 1 aufgeführten Derivate hergestellt, nach bekannter Verfahrensweise zu Flachmembranen verarbeitet und untersucht.

Beispiel 10

In einem Mischer wurden 36,8 g (0,4 Mol) Thioglykolsäure vorgelegt und mit einer Lösung von 44,8 g (0,8 Mol) Kaliumhydroxid in 60 ml Wasser neutralisiert. Danach wurden 37,94 g (0,2 Mol) des Esters vom Beispiel 1 A hinzugefügt und das Gemisch 24 Stunden bei 70 °C erhitzt. Das Reaktionsprodukt wurde mit Wasser und Äthanol gewaschen und im Vakuumtrockenschrank bei 65 °C getrocknet. Dabei wurden 32,7 g eines Produktes mit einem Kaliumgehalt von 2,09%, entsprechend einem DS(n) von 0,093, erhalten.

Aus diesem Cellulosederivat wurde nach üblicher Verfahrensweise eine Cuoxamlösung mit 9% Cellulosederivatgehalt hergestellt und zu Flachmembranen verarbeitet.

Im Vergleich zu unmodifizierter Cellulose beträgt die C5a-Reduktion 99%.

Beispiele 11 - 20

Analog dem Beispiel 1, 2, 3 oder 10 wurden die in der Tabelle 2 aufgeführten Derivate synthetisiert, nach bekannter Verfahrensweise zu Flachmembranen verarbeitet und ihre Komplementaktivierung anhand der Fragmente C5a bestimmt.

**Tabelle 1**

| Beispiel | Ausgangs-polymer | s | X' | Y' | Z | T | X | Y | n | C3a-Redukt. % | ß-2-μ-globulin-ads. % |
|---|---|---|---|---|---|---|---|---|---|---|---|
| 4 | Cellulose | - | - | - | S | - | $CH_2$ | COOH | 0,12 | 94 | 25 |
| 5 | Cellulose | - | - | - | S | - | $C_2H_4$ | $SO_3H$ | 0,07 | 96 | 33 |
| 6 | Cellulose | 0,03 | $C_2H_4$ | $SO_3H$ | N | H | $CH_2$ | COOH | 0,04 | 98 | 43 |
| 7 | Cellulose | - | - | - | N | H | $(CH_3)_2C_2H_2$ | COOH | 0,06 | 89 | 32 |
| 8 | Cellulose | 0,04 | $C_2H_4$ | $SO_3H$ | S | – | $CH_2$ | COOH | 0,09 | 99 | 56 |
| 9 | Chitin | - | - | - | S | – | $CH_2$ | COOH | 0,13 | 87 | 47 |

**Tabelle 2**

| Beispiel | Ausgangs-polymer | s | X'-Y' | Z | T | X - Y | n | C5a-Red % |
|---|---|---|---|---|---|---|---|---|
| 11 | Cellulose | - | - | S | - | $CH_2$-COOH | 0,03 | 98 |
| 12 | Cellulose | - | - | S | - | $C_6H_{13}$ | 0,30 | 90 |
| 13 | Cellulose | - | - | S | - | $C_6H_5$ | 0,20 | 70 |
| 14 | Cellulose | - | - | S | - | $C_2H_4$-OH | 0,15 | 65 |
| 15 | Cellulose | - | - | S | - | $C_3H_6$-$SO_3H$ | 0,06 | 87 |
| 16 | Cellulose | - | - | N | H | $C_6H_3$(-OH)-COOH | 0,10 | 92 |
| 17 | Cellulose | 0,05 | N(i-$C_3H_7$)$_2$ | S | - | $CH_2$COOH | 0,08 | 100 |
| 18 | Cellulose | 0,07 | $C_5H_{10}N$ | N | H | CH(-COOH)-$C_6H_5$ | 0,03 | 100 |
| 19 | Cellulose | 0,12 | $C_4H_8$-$SO_3H$ | S | - | $C_{12}H_{25}$ | 0,02 | 85 |
| 20 | Cellulose | 0,08 | $C_7H_{15}$-COOH | S | - | $C_6H_5$ | 0,03 | 90 |

## Patentansprüche

1. Dialysemembran für die Hämodialyse in Form von Flachfolien, Schlauchfolien oder Hohlfäden aus durch Substitution modifizierter Cellulose, dadurch gekennzeichnet, daß die modifizierte Cellulose eine

durch die Formel

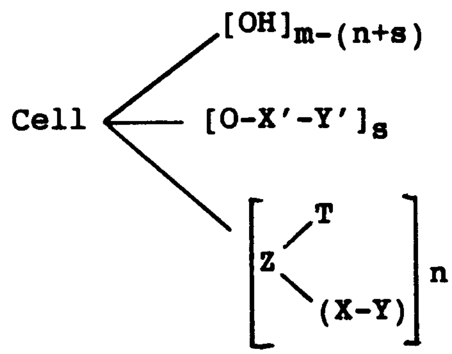

wiedergegebene Struktur aufweist, worin Cell das Gerüst des unmodifizierten Cellulosemoleküls oder des Chitinmoleküls jeweils ohne Hydroxylgruppen, Z ein N- oder S-Atom ist und für N T ein Wasserstoffatom oder X-Y bedeutet und für S T entfällt und (n + s) den mittleren Substitutionsgrad angibt mit $0 < n < m$ und $0 \leqq s < m$ und $n + s < m$ und m beim unmodifizierten Cellulosemolekül 3 und beim Chitinmolekül 2 beträgt und worin gegebenenfalls - X - entfallen kann oder

-X- und -X' - einen gegebenenfalls substituierten Alkylen-Rest (gerad-kettig und/oder verzweigt, wobei die Kohlenstoffkette auch durch Heteroatome wie O, S, N, unterbrochen sein kann) und/oder Arylalkylen-, Aryl-Rest (ggf. mit Heteroatomen und/oder substituiert)

-Y und -Y' -H und/oder $-NR_2$ und/oder -COOH auch als Salz und/oder $-SO_3H$ auch als Salz und/oder -OR bedeuten,

wobei R ein Wasserstoffatom und/oder eine gegebenenfalls substituierte Alkyl-, (gerad-kettig und/oder verzweigt, wobei die Kohlenstoffkette auch durch Heteroatome wie O, S, N unterbrochen sein kann) und/oder Aryl- und/oder Arylalkyl- (ggf. mit Heteroatomen und/oder substituiert) bedeutet

und X gleich oder verschieden von X' und Y gleich oder verschieden von Y' ist und die maximale Anzahl der C-Atome in X-Y bzw. X'-Y' 12 beträgt.

**2.** Dialysemembran nach Anspruch 1, dadurch gekennzeichnet, daß n = 0,02 bis 0,30 beträgt.

**3.** Dialysemembran nach einem oder mehreren der Ansprüche 1 bis 2, dadurch gekennzeichnet, daß s = 0,03 bis 0,12 beträgt.

**4.** Dialysemembran nach einem oder mehreren der Ansprüche 1 bis 3, dadurch gekennzeichnet, daß X bzw. X' mit den Molekülresten Y bzw. Y' substituiert ist.

**5.** Dialysemembran nach einem oder mehreren der Ansprüche 1 bis 4, dadurch gekennzeichnet, daß die modifizierte Cellulose im Substituenten sekundäre, tertiäre oder quaternäre Aminogruppen, Carboxygruppen und/oder Sulfogruppen enthält.

**6.** Dialysemembran nach Anspruch 5, dadurch gekennzeichnet, daß -[ XY ] Dialkylaminoalkylen, Carboxyalkylen, Carboxyarylalkylen und/oder Sulfoalkylen bedeutet.

**7.** Dialysemembran nach einem oder mehreren der Ansprüche 1 bis 6, dadurch gekennzeichnet, daß die Alkylgruppen im Substituenten Äthylgruppen, Methylgruppen und/oder Propylgruppen sind.

## Claims

**1.** Dialysis membrane for haemodialysis in the form of flat films, tubular films or hollow threads of cellulose modified by substitution, characterised in that the modified cellulose has a structure represented by the following formula

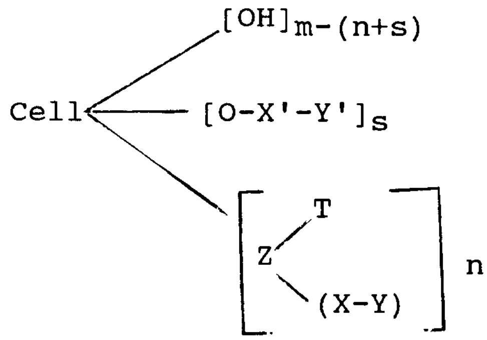

wherein Cell stands for the structure of the unmodified cellulose molecule or of the chitin molecule, in each case without hydroxyl groups, Z stands for an N- or S-atom and when Z stands for N, T denotes a hydrogen atom or X-Y and when Z stands for S, T is absent, and (n + s) indicates the average degree of substitution, where $0 < n < m$ and
$0 \leqq s < m$ and $n + s < m$ and the value of m is 3 in the unmodified cellulose molecule and 2 in the chitin molecule and - X - may optionally be absent or

-X- and -X'- denote an optionally substituted alkylene group (straight chain and/or branched, and the carbon chain may also be interrupted by hetero-atoms such as O, S or N) and/or an arylalkylene or aryl group (optionally containing hetero-atoms and/or substituted),

-Y and -Y' stand for H and/or $-NR_2$ and/or-COOH, optionally in the form of a salt, and/or $-SO_3H$, optionally in the form of a salt, and/or -OR,

where R denotes a hydrogen atom and/or an optionally substituted alkyl group (straight chain and/or branched and the carbon chain may also be interrupted by hetero-atoms such as O, S or N) and/or aryl and/or arylalkyl (optionally containing hetero-atoms and/or substituted)

and X may be identical to or different from X', Y may be identical to or different from Y' and the maximum number of carbon atoms in X-Y or X'-Y' is 12.

2. Dialysis membrane according to Claim 1, characterised in that n = 0.02 to 0.30.

3. Dialysis membrane according to one or more of Claims 1 to 2, characterised in that s = 0.03 to 0.12.

4. Dialysis membrane according to one or more of Claims 1 to 3, characterised in that X or X' is substituted with the molecular radicals Y or Y'.

5. Dialysis membrane according to one or more of Claims 1 to 4, characterised in that the modified cellulose contains secondary, tertiary or quaternary amino groups, carboxyl groups and/or sulpho groups in the substituent.

6. Dialysis membrane according to Claim 5, characterised in that -[ XY ] denotes dialkylamino alkylene, carboxyalkylene, carboxyarylalkylene and/or sulphoalkylene.

7. Dialysis membrane according to one or more of Claims 1 to 6, characterised in that the alkyl groups in the substituent are ethyl groups, methyl groups and/or propyl groups.

## Revendications

1. Membrane de dialyse pour hémodialyse, se présentant sous la forme de feuilles planes, de feuilles tubulaires ou de fibres creuses, en cellulose modifiée par substitution, caractérisée en ce que la cellulose modifiée possède une structure représentée par la formule :

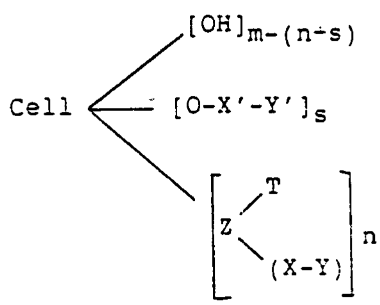

où "Cell" représente le squelette de la molécule de cellulose non modifiée ou de la molécule de chitine, dans chaque cas sans les groupes hydroxyles, Z représente un atome d'azote ou de soufre et T représente un atome d'hydrogène ou -X-Y, dans le cas de l'azote, ou disparait dans le cas du soufre, et (n + s) indique le degré moyen de substitution, avec $0 < n < m$ et $0 \leqq s < m$ et $n + s < m$, m valant 3 dans le cas de la molécule de cellulose non modifiée et 2 dans le cas de la molécule de chitine, et où -X- peut éventuellement disparaitre ou
-X- et -X'- représentent un groupe alkylène éventuellement substitué (à chaîne droite et/ou ramifiée, la chaîne d'atomes de carbone pouvant aussi être interrompue par des hétéroatomes comme O, S et N) et/ou un groupe arylalkylène ou aryle (le cas échéant, substitué et/ou comportant des hétéroatomes), et
-Y et -Y' représentent -H et/ou $-NR_2$ et/ou -COOH (même salifié) et/ou $-SO_3H$ (même salifié) et/ou -OR,
R représentant un atome d'hydrogène et/ou un groupe alkyle éventuellement substitué (à chaîne droite et/ou ramifiée, la chaîne d'atomes de carbone pouvant aussi être interrompue par des hétéroatomes comme O, S et N) et/ou un groupe arylalkyle et/ou aryle (le cas échéant, substitué et/ou comportant des hétéroatomes),
X et X' étant identiques ou différents, Y et Y' étant identiques ou différents, et le nombre d'atomes de carbone dans -X-Y ou dans -X'-Y' valant au maximum 12.

**2.** Membrane de dialyse selon la revendication 1, caractérisée en ce que n vaut de 0,02 à 0,30.

**3.** Membrane de dialyse selon une ou plusieurs des revendications 1 et 2, caractérisée en ce que s vaut de 0,03 à 0,12.

**4.** Membrane de dialyse selon une ou plusieurs des revendications 1 à 3, caractérisée en ce que X ou X' est substitué par un groupe Y ou Y', respectivement.

**5.** Membrane de dialyse selon une ou plusieurs des revendications 1 à 4, caractérisée en ce que la cellulose modifiée comporte, parmi ses substituants, des groupes amino secondaires, tertiaires ou quaternaires, des groupes carboxy et/ou des groupes sulfo.

**6.** Membrane de dialyse selon la revendication 5, caractérisée en ce que -(XY) représente un groupe dialkylaminoalkylène, carboxyalkylène, carboxyarylalkylène et/ou sulfoalkylène.

**7.** Membrane de dialyse selon une ou plusieurs des revendications 1 à 6, caractérisée en ce que, dans les substituants, les groupes alkyle sont des groupes éthyle, méthyle et/ou propyle.